# EUROPEAN PATENT SPECIFICATION

(11) **EP 3 335 265 B1**
(45) Date of publication and mention of the grant of the patent: **26.02.2020**
(21) Application number: 16748320.5
(22) Date of filing: 10.08.2016
(51) Int. Cl.: H01M 10/052, H01M 10/0567

(54) **CYCLIC DINITRILE COMPOUNDS AS ADDITIVES FOR ELECTROLYTE**
CYCLISCHE DINITRILVERBINDUNGEN ALS ADDITIVE FÜR ELEKTROLYT
COMPOSÉS DINITRILES CYCLIQUE COMME ADDITIFS D'ÉLECTROLYTE

(30) Priority: 14.08.2015 EP 15181137
(43) Date of publication of application: 20.06.2018
(73) Proprietor: BASF SE, 67056 Ludwigshafen am Rhein (DE)
(72) Inventor: CHESNEAU, Frederick Francois, 68789 St. Leon-Rot (DE); SCHMIDT, Michael, 64665 Alsbach-Haehnlein (DE); GASPAR, Boris, 70174 Stuttgart (DE); MERGER, Martin, 67227 Frankenthal (DE)
(74) Representative: BASF IP Association
(86) International application number: PCT/EP2016/069078
(87) International publication number: WO 2017/029176

(56) References cited:
- WO-A1-2015/007554
- JP-A- 2013 026 042
- US-A1- 2006 194 118
- EVA H. MØRKVED ET AL: "Preparation of Octa(alkoxy) Azaphthalocyanines.", ACTA CHEMICA SCANDINAVICA., vol. 53, 1 January 1999 (1999-01-01), pages 1117-1121, XP055220895, DK ISSN: 0904-213X, DOI: 10.3891/acta.chem.scand.53-1117
- USLU KOBAK R Z ET AL: "The synthesis and cyclotetramerisation reactions of aryloxy-, arylalkyloxy-substituted pyrazine-2,3-dicarbonitriles and spectroelectrochemical properties of octakis(hexyloxy)-pyrazinoporphyrazine", DYES AND PIGMENTS, ELSEVIER APPLIED SCIENCE PUBLISHERS. BARKING, GB, vol. 86, no. 2, 1 July 2010 (2010-07-01), pages 115-122, XP026907096, ISSN: 0143-7208, DOI: 10.1016/J.DYEPIG.2009.12.003 [retrieved on 2010-02-15]
- HASRAT ALI ET AL: "An easy route for the synthesis of pyrazine-2,3-dicarbonitrile 5,6-bis-substituted derivatives using a palladium catalyst", TETRAHEDRON LETTERS, vol. 53, no. 36, 1 September 2012 (2012-09-01), pages 4824-4827, XP055220940, GB ISSN: 0040-4039, DOI: 10.1016/j.tetlet.2012.06.102
- VERONIKA NOVAKOVA ET AL: "Role of Steric Hindrance in the Newman-Kwart Rearrangement and in the Synthesis and Photophysical Properties of Arylsulfanyl Tetrapyrazinoporphyrazines", THE JOURNAL OF ORGANIC CHEMISTRY, vol. 79, no. 5, 7 March 2014 (2014-03-07), pages 2082-2093, XP055220923, US ISSN: 0022-3263, DOI: 10.1021/jo402791c
- EVA H. MØRKVED ET AL: "Preparation of Magnesium Azaphthalocyanines by Cyclotetramerisation of S-Substituted 4,5-Disulfanylpyrazine-2,3-dicarbonitriles .", ACTA CHEMICA SCANDINAVICA., vol. 50, 1 January 1996 (1996-01-01), pages 1153-1156, XP055220928, DK ISSN: 0904-213X, DOI: 10.3891/acta.chem.scand.50-1153
- TURCHI S ET AL: "Hetero Diels-Alder Reactions of 4,5-Dicyanopyridazine with Alkenes", TETRAHEDRON, ELSEVIER SCIENCE PUBLISHERS, AMSTERDAM, NL, vol. 53, no. 34, 25 August 1997 (1997-08-25), pages 11711-11720, XP004106033, ISSN: 0040-4020, DOI: 10.1016/S0040-4020(97)00737-0

## Description

The present invention relates to cyclic dinitrile compounds of formula (I) 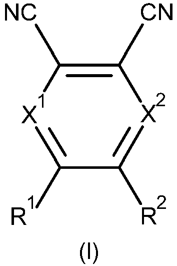 wherein X¹, X², R¹, and R² are defined as described below and to their use in electrolyte compositions and electrochemical cells.

Storing electrical energy is a subject of still growing interest. Efficient storage of electric energy would allow electric energy to be generated when it is advantageous and used when needed. Secondary electrochemical cells are well suited for this purpose due to their reversible conversion of chemical energy into electrical energy and vice versa (rechargeability). Secondary lithium batteries are of special interest for energy storage since they provide high energy density and specific energy due to the small atomic weight of the lithium ion, and the high cell voltages that can be obtained (typically 3 to 5 V) in comparison with other battery systems. For that reason, these systems have become widely used as a power source for many portable electronics such as cellular phones, laptop computers, mini-cameras, etc.

Secondary lithium batteries like lithium ion batteries typically comprise electrolyte compositions containing one or more organic aprotic solvents, e.g. non-aqueous solvents like organic carbonates, ethers, esters and ionic liquids, at least one conducting salt like LiPF₆ and optionally one or more additives for enhancing the performance of electrolyte composition and battery. Useful additives are for example SEI additives, flame retardant additives, water scavenger, overcharge protection additives. A lot of research is ongoing in respect to additives for use in electrolyte compositions to further improve the performance of the electrochemical cell containing the electrolyte composition in many different aspects, e.g. cycle life time, high temperature characteristics, safety, etc.

WO 2015/007554 A1 discloses the use of cyclic dinitriles as additives in electrolyte compositions to obtain lithium secondary ion batteries showing improved capacity retention.

E.H. Mørkved et al., Acta Chemica Scandinavica Vol. 53 (1999), pages 1117-1121 describes the synthesis of octa(alkoxy) azaphthalocyanines, wherein 5,6-dialkoxypyrazin-2,3-dicarbonitriles are used as intermediates.

R.Z.U. Kobak et al., Dyes and Pigments Vol. 86 (2010), pages 115-122 refers to the synthesis and cyclotetramerization reactions of aryloxy- and arylalkyloxy-substituted pyrazine-2,3-dicarbonitriles.

H. Ali et al., Tetrahedron Letters Vol. 53 (2012), pages 4824-4827 describes the synthesis of pyrazine-2,3-dicarbonitrile 5,6-bis-substituted derivatives.

V. Novakova et al., The Journal of Organic Chemistry Vol. 79 (2014), pages 2082-2093 investigates inter alia the role of steric hindrance in the synthesis of arylsulfanyl tetrapyrazinoporphyrazines wherein 5,6-bis(phenylsulfanyl)pyrazin-2,3-dicarbonitrile is used as intermediate.

E.H. Mørkved et al., Acta Chemica Scandinavica Vol. 50 (1996), pages 1153-1156 describes the synthesis of magnesium azaphthalocyanindes by cyclotetramerization of S-substituted 4,5-disulfanylpyrazine-2,3-dicarbonitriles.

S. Turchi et al. Tetrahedron Vol. 53 (1997), pages 11711-11720 describes hetero Diels-Alder reactions of 4,5-dicyanopyridazine with alkenes which result inter alia in 1,2-dicarbonitrilbenzenes substituted in 4,5-position by two ring forming substituents.

JP 2013-026042 A discloses electrolytes for lithium batteries comprising compounds selected inter alia from alkoxylated 1,2-dicarbonitrilbenzene.

US 2006/0194118 A1 describes electrolyte compositions for lithium batteries comprising additives which are selected inter alia from 1,2-dicarbonitrilbenzene.

Further aspects of the performance of electrochemical cells are the internal resistance, the increase of internal resistance over the lifetime of the battery, the gas evolution over the life of the battery, and the dissolution of transition metal ions present in the electrode active materials of electrochemical cells. There is still the need for additives for electrolyte compositions leading to improved battery performance, inter alia in respect to the aforementioned aspects. It is in particular desirable if such additives combine more than one favorable property and/or at least do not have a detrimental effect on other properties of the electrochemical cells in which they are used.

It was an object of the present invention to provide compounds suited as additives for electrolyte compositions yielding secondary batteries with improved properties like low gas evolution and low transition metal dissolution from the electrode active materials. A further object of the present invention was to provide secondary batteries of high energy density and/or higher operating voltage having good performance characteristics.

These objects are achieved by electrolyte compositions containing at least one conducting salt selected from lithium salts and at least one compound of formula (I) 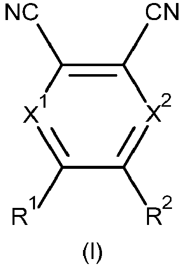 wherein
X¹ and X² are N;
R¹ and R² are selected independently from each other from R⁴, OR⁴, OSi(R⁸)₃, SR⁴, S(O)R⁴, S(O)₂R⁴, OS(O)₂R⁴, S(O)₂OR⁴, and NR⁵R⁶ ;
R⁴ is selected from C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F, CN, C(O)OR⁷, OC(O)R⁷, OR⁷, and SR⁷;
R⁵ and R⁶ are selected independently from each other from H, C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
or R⁵ and R⁶ may be combined to form together with the C- or N-atom a 5- to 7-membered heterocycle which may be substituted by one or more substituents selected from F, CN, C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
R⁷ is selected from C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl which may be substituted by one or more substituents selected from F and CN; and
R⁸ is independently selected from R⁴ and OR⁴;
and the use of compounds of formula (I) in electrolyte compositions for electrochemical cells.

The problem is further solved by electrochemical cells comprising said electrolyte composition.

The addition of at least one compound of formula (I) to an electrolyte composition for rechargeable electrochemical cells leads to less gassing, comparably low impedance and may lead to a significant reduction of transition metal dissolution in electrochemical cells.

In the following the invention is described in detail.

The electrolyte composition contains at least one compound of formula (I) 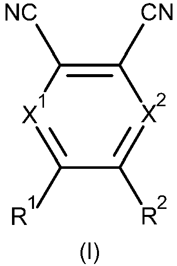
X¹ and X² are selected independently from each other from N;
R¹ and R² are selected independently from each other from R⁴, OR⁴, SR⁴, S(O)R⁴, S(O)₂R⁴, OS(O)₂R⁴, S(O)₂OR⁴, OSiR⁸₃, and NR⁵R⁶;
R⁴ is selected from C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F, CN, C(O)OR⁷, OC(O)R⁷, OR⁷, and SR⁷;
R⁵ and R⁶ are selected independently from each other from H, C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
or R⁵ and R⁶ may be combined to form together with the C- or N-atom a 5- to 7-membered heterocycle which may be substituted by one or more substituents selected from F, CN, C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
R⁷ is selected from C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl which may be substituted by one or more substituents selected from F and CN; and
R⁸ is independently selected from R⁴ and OR⁴.

The term "C₁ to C₁₂ alkyl" as used herein means a straight or branched saturated hydrocarbon group with 1 to 12 carbon atoms having one free valence and wherein one or more CH₂-groups may be replaced by O or S, e.g., methyl, ethyl, n-propyl, isopropyl, n-butyl, isobutyl, tert-butyl, n-pentyl, iso-pentyl, 2,2-dimethylpropyl, n-hexyl, iso-hexyl, 2-ethyl hexyl, n-heptyl, iso-heptyl, n-octyl, iso-octyl, n-nonyl, n-decyl, methoxymethyl, ethoxymethyl, methoxyethyl, and the like. Preferred are C₁-C₆ alkyl, more preferred are C₁-C₄ alkyl groups and most preferred are methyl, ethyl, and n- and iso-propyl.

The term "C₃ to C₆ (hetero)cycloalkyl" as used herein means a saturated 3- to 6-membered hydrocarbon cycle having one free valence wherein one or more of the C- atoms of the saturated cycle may be replaced independently from each other by a heteroatom selected from N, S, O and P. Examples of C₃ to C₆ cycloalkyl include cyclopropyl, cyclobutyl, cyclopentyl, and cyclohexyl, preferred is cyclohexyl. Examples of C₃ to C₆ hetero cycloalkyl are oxiranyl, tetrahydrofuryl, pyrrolidyl, piperidyl and morpholinyl.

The term "C₂ to C₁₂ alkenyl" as used herein refers to an unsaturated straight or branched hydrocarbon group with 2 to 12 carbon atoms having one free valence. Unsaturated means that the alkenyl group contains at least one C-C double bond and wherein one or more CH₂-groups may be replaced by O or S. C₂ to C₁₂ alkenyl includes for example ethenyl, 1-propenyl, 2-propenyl, 1-n-butenyl, 2-n-butenyl, iso-butenyl, 1-pentenyl, 1-hexenyl, 1-heptenyl, 1-octenyl, 1-nonenyl, 1-decenyl, methoxyethenyl, and the like. Preferred are C₂ to C₈ alkenyl groups, more preferred are C₂ to C₆ alkenyl groups, even more preferred are C₂ to C₄ alkenyl groups and in particular ethenyl and propenyl, the preferred propenyl is 1-propen-3-yl, also called allyl.

The term "C₂ to C₁₂ alkynyl" as used herein refers to an unsaturated straight or branched hydrocarbon group with 2 to 12 carbon atoms having one free valence, wherein the hydrocarbon group contains at least one C-C triple bond and wherein one or more CH₂-groups may be replaced by O or S. C₂ to C₁₀ alkynyl includes for example ethynyl, 1-propynyl, 2-propynyl, 1-n-butinyl, 2-n-butynyl, 1-pentynyl, 1-hexynyl, -heptynyl, 1-octynyl, 1-nonynyl, 1-decynyl, methoxyethynyl, and the like. Preferred are C₂ to C₁₀ alkynyl, more preferred are C₂ to C₆ alkynyl, even more preferred are C₂ to C₄ alkynyl, in particular preferred are ethynyl and 1-propyn-3-yl (propargyl).

The term "C₅ to C₁₂ (hetero)aryl" as used herein denotes an aromatic 5- to 12-membered hydrocarbon cycle or condensed cycles having one free valence wherein one or more of the C- atoms of the aromatic cycle(s) may be replaced independently from each other by a heteroatom selected from N, S, O and P. Examples of C₅-C₁₂ (hetero)aryl are pyrrolyl, furanyl, thiophenyl, pyridinyl, pyranyl, thiopyranyl, phenyl, and naphtyl. Preferred is phenyl.

The term "C₆ to C₂₄ (hetero)aralkyl" as used herein denotes an aromatic 5- to 12-membered hydrocarbon cycle substituted by one or more C₁ to C₆ alkyl wherein one or more of the C-atoms of the aromatic cycle may be replaced independently from each other by a heteroatom selected from N, S, O and P and one or more CH₂-groups of the alkyl may be substituted by O or S. The C₆ to C₂₄ (hetero)aralkyl group contains in total 6 to 24 C- and heteroatoms and has one free valence. The free valence may be located in the aromatic cycle or in a C₁ to C₆ alkyl group, i.e. C₆ to C₂₄ (hetero)aralkyl group may be bound via the (hetero)aromatic part or via the alkyl part of the group. Examples of C₆-C₂₄ (hetero)aralkyl are methylphenyl, 2-methylpyridyl, 1,2-dimethylphenyl, 1,3-dimethylphenyl, 1,4-dimethylphenyl, ethylphenyl, 2-propylphenyl, benzyl, CH₂-pyridyl, and the like, preferred is benzyl
R¹ and R² are selected independently from each other from R⁴, OR⁴, SR⁴, S(O)R⁴, S(O)₂R⁴, OS(O)₂R⁴, S(O)₂OR⁴, OSiR⁸₃, and NR⁵R⁶; preferably R¹ and R² are selected from R⁴, OR⁴, SR⁴, OSiR⁸₃, and NR⁵R⁶, and most preferred R¹ and R² are selected from OR⁴ and SR⁴, wherein R⁴ is selected from C₁ to C₁₂ alkyl, C₅ to C₁₂ (hetero)aryl and C₆ to C₂₄ (hetero)aralkyl which may be substituted by one or more substituents selected from F, CN, C(O)OR⁷, OC(O)R⁷, OR⁷, and SR⁷, preferably R⁴ is selected from C₅ to C₁₂ (hetero)aryl and C₆ to C₂₄ (hetero)aralkyl which may be substituted by one or more substituents selected from F, CN, C(O)OR⁷, OC(O)R⁷, OR⁷, and SR⁷; or R¹ and R² form together with the C-C bond a 5- to 7-membered unsaturated cycle, which may be substituted by one or more groups selected from F, CN, R⁴, OR⁴, SR⁴, S(O)R⁴, S(O)₂R⁴, OS(O)₂R⁴, S(O)₂OR⁴, OSiR⁸₃, and NR⁵R⁶.

According to one embodiment of the invention R¹ and R² are selected independently from each other from OR⁴, SR⁴, S(O)₂R⁴, OS(O)₂R⁴, S(O)₂OR⁴, OSiR⁸₃, and NR⁵R⁶preferably R¹ and R² are selected independently from each other from OR⁴, SR⁴, S(O)₂R⁴, OS(O)₂R⁴, S(O)₂OR⁴, OSiR⁸₃, and NR⁵R⁶, and more preferred R¹ and R² are selected independently from each other from OR⁴, SR⁴, and NR⁵R⁶.

According to another embodiment of the invention R¹ and R² are selected independently from each other selected from R⁴.

It is preferred in any case, that R¹ and R² are equal.

R⁴ is selected from C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F, CN, C(O)OR⁷, OC(O)R⁷, OR⁷, and SR⁷; preferably R⁴ is selected from C₁ to C₁₂ alkyl, C₅ to C₁₂ (hetero)aryl, and C₆ to C₂₄ (hetero)aralkyl wherein alkyl, (hetero)aryl, and hetero(aralkyl) may be substituted by one or more substituents selected from F, CN, C(O)OR⁷, OC(O)R⁷, OR⁷, and SR⁷.

R⁵ and R⁶ are selected independently from each other from H, C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN; preferably R⁵ and R⁶ are selected independently from each other from H, C₁ to C₁₂ alkyl, C₅ to C₁₂ (hetero)aryl, and C₆ to C₂₄ (hetero)aralkyl which may be substituted by one or more substituents selected from F and CN;
or R⁵ and R⁶ may be combined to form together with the C- or N-atom a 5- to 7-membered heterocycle which may be substituted by one or more substituents selected from F, CN, C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN; preferably 5- to 7-membered heterocycle may be substituted by F, CN, and C₁ to C₁₂ alkyl.

R⁷ is selected from C₁ to C₁₂ alkyl, C₃ to C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl which may be substituted by one or more substituents selected from F and CN, preferably R⁷ is selected from C₁ to C₁₂ alkyl, which may be substituted by one or more substituents selected from F and CN.

R⁸ is independently selected from R⁴ and OR⁴, e.g. in the group OSiR⁸₃ all R⁸ may be OR⁴, or all R⁸ may be R⁴ or OSiR⁸₃ may contain both OR⁴ and R⁴. Preferably R⁸ is selected from OC₁ to C₁₂ alkyl and C₁ to C₁₂ alkyl, most preferred R⁸ is selected from OC₁ to C₁₂ alkyl.

Preferred compounds of formula (I) are compounds of formula (I) wherein
X¹ and X² are N,
R¹ and R² are selected independently from each other from R⁴, OR⁴, and SR⁴, preferably from OR4, and SR⁴, and
R⁴ is selected from C₁ to C₁₂ alkyl, C₅ to C₁₂ (hetero)aryl, and C₆ to C₂₄ (hetero)aralkyl wherein alkyl, (hetero)aryl, and hetero(aralkyl) may be substituted by one or more substituents selected from F and CN.

Examples of compounds of formula (I) are compounds of formula
(I.a) X¹ and X² are N and R¹ and R² are O-CH₂-phenyl or O-CH₂-CF₃,
(I.b) X¹ and X² are N and R¹ and R² are S-phenyl or S-CH₂-Phenyl,
(I.c) X¹ and X² are N and R¹ and R² are phenyl or ethynyl, and
(I.d) X¹ and X² are N and R¹ and R² are OCH₃.

The preparation of the compounds of formula (I) is known to the person skilled in the art. For example they may be prepared by reacting diaminonitrile with R¹C(O)C(O)R², or by reacting 5,6-dichloro-2,3-dicyanopyrazine with the respective nucleophiles Nu¹-R¹ and Nu²-R².

The reaction of diaminonitrile with R¹C(O)C(O)R² is a condensation reaction according to scheme (a):

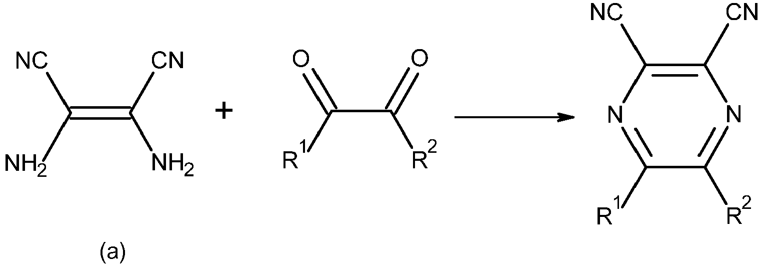

The reaction of 5,6-dichloro-2,3-dicyanopyrazine with nucleophiles like alcohols, thiols, amine, organometallic reagents etc. follows scheme (b): 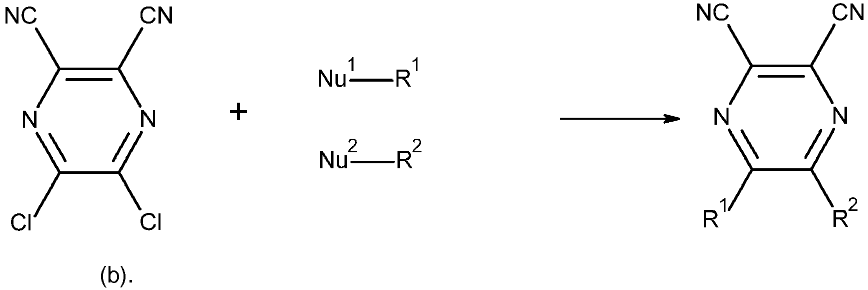 Nu¹-R¹ and Nu²-R² are the nucleophilic derivatives of R¹ and R², respectively, which allow the reaction with the aromatic dichloro compound to yield the cyclic dinitriles of formula (I). E.g. Nu¹-R¹ and Nu²-R² may be selected from R⁴OH, R⁴SH, HNR⁵R⁶, , R⁴-S(O)₂-OH, R⁴-S(O)₂-OM (M=Li, Na, K), and nucleophiles functionalized by an organometallic groups M*-R¹ and M*-R² like Aryl-MgBr or Aryl-MgCI, vinyl-MgBr, ethynyl-MgBr. Compounds of formula (I) wherein R¹ and/or R² are selected from S(O)₂OR⁴ may be prepared by oxidation of the reaction product of R⁴SH with the dichloro compound. The preparation of the compounds of formula (I) described herein is also an object of the present invention.

According to another object of the invention the compounds of formula (I), as described above or as described as being preferred, are used in electrolyte compositions for electrochemical cells. It is preferred to use the compounds of formula (I) in non-aqueous electrolyte compositions, more preferred the compounds of formula (I) are used in electrolyte compositions for lithium batteries, even more preferred in electrolyte compositions for lithium ion batteries.
Accordingly, when a compound of formula (I) is used in an electrolyte composition, the total concentration of the compound(s) of formula (I) in the electrolyte composition is typically 0.005 to 10 wt.-%, preferred 0.01 to 5 wt.-% and most preferred 0.05 to 2 wt.-%, based on the total weight of the electrolyte composition. Usually the compound(s) of formula (I) are added to the electrolyte composition in the desired amount during or after manufacture of the electrolyte composition.

A further object of the invention is an electrolyte composition containing at least one compound of formula (I). The minimum total concentration of compounds of formula (I) in the electrolyte composition is usually 0.005 wt.-%, typically the total concentration of compound(s) of formula (I) in the electrolyte composition is 0.005 to 10 wt.-%, preferred 0.01 to 5 wt.-% and most preferred 0.05 to 2 wt.-%, based on the total weight of the electrolyte composition. The term "wt.-%" as used herein means percent by weight.

The electrolyte composition preferably contains at least one aprotic organic solvent, more preferred at least two aprotic organic solvents. According to one embodiment the electrolyte composition may contain up to ten aprotic organic solvents.

The at least one aprotic organic solvent is preferably selected from cyclic and acyclic organic carbonates, di-C₁-C₁₀-alkylethers, di-C₁-C₄-alkyl-C₂-C₆-alkylene ethers and polyethers, cyclic ethers, cyclic and acyclic acetales and ketales, orthocarboxylic acids esters, cyclic and acyclic esters of carboxylic acids, cyclic and acyclic sulfones, and cyclic and acyclic nitriles and dinitriles.

More preferred the at least one aprotic organic solvent is selected from cyclic and acyclic carbonates, di-C₁-C₁₀-alkylethers, di-C₁-C₄-alkyl-C₂-C₆-alkylene ethers and polyethers, cyclic and acyclic acetales and ketales, and cyclic and acyclic esters of carboxylic acids, even more preferred the electrolyte composition contains at least one aprotic organic solvent selected from cyclic and acyclic carbonates, and most preferred the electrolyte composition contains at least two aprotic organic solvents selected from cyclic and acyclic carbonates, in particular preferred the electrolyte composition contains at least one aprotic solvent selected from cyclic carbonates and at least one aprotic organic solvent selected from acyclic carbonates.

The aprotic organic solvents may be partly halogenated, e.g. they may be partly fluorinated, partly chlorinated or partly brominated, and preferably they may be partly fluorinated. "Partly halogenated" means, that one or more H of the respective molecule is substituted by a halogen atom, e.g. by F, Cl or Br. Preference is given to the substitution by F. The at least one solvent may be selected from partly halogenated and non-halogenated aprotic organic solvents i.e. the electrolyte composition may contain a mixture of partly halogenated and non-halogenated aprotic organic solvents.

Examples of cyclic carbonates are ethylene carbonate (EC), propylene carbonate (PC) and butylene carbonate (BC), wherein one or more H of the alkylene chain may be substituted by F and/or an C₁ to C₄ alkyl group, e.g. 4-methyl ethylene carbonate, monofluoroethylene carbonate (FEC), and cis- and trans-difluoroethylene carbonate. Preferred cyclic carbonates are ethylene carbonate, monofluoroethylene carbonate and propylene carbonate, in particular ethylene carbonate.

Examples of acyclic carbonates are di-C₁-C₁₀-alkylcarbonates, wherein each alkyl group is selected independently from each other, preferred are di-C₁-C₄-alkylcarbonates. Examples are e.g. diethyl carbonate (DEC), ethyl methyl carbonate (EMC), dimethyl carbonate (DMC), and methylpropyl carbonate. Preferred acyclic carbonates are diethyl carbonate (DEC), ethyl methyl carbonate (EMC), dimethyl carbonate (DMC).

In one embodiment of the invention the electrolyte composition contains mixtures of acyclic oganic carbonates and cyclic organic carbonates at a ratio by weight of from 1:10 to 10:1, preferred of from 5:1 to 1:5.

According to the invention each alkyl group of the di-C₁-C₁₀-alkylethers is selected independently from the other. Examples of di-C₁-C₁₀-alkylethers are dimethylether, ethyl-methylether, diethylether, methylpropylether, diisopropylether, and di-n-butylether.

Examples of di-C₁-C₄-alkyl-C₂-C₆-alkylene ethers are 1,2-dimethoxyethane, 1,2-diethoxyethane, diglyme (diethylene glycol dimethyl ether), triglyme (triethyleneglycol dimethyl ether), tetraglyme (tetraethyleneglycol dimethyl ether), and diethylenglycoldiethylether.

Examples of suitable polyethers are polyalkylene glycols, preferably poly-C₁-C₄-alkylene glycols and especially polyethylene glycols. Polyethylene glycols may comprise up to 20 mol% of one or more C₁-C₄-alkylene glycols in copolymerized form. Polyalkylene glycols are preferably dimethyl- or diethyl- end-capped polyalkylene glycols. The molecular weight M_{w} of suitable polyalkylene glycols and especially of suitable polyethylene glycols may be at least 400 g/mol. The molecular weight M_{w} of suitable polyalkylene glycols and especially of suitable polyethylene glycols may be up to 5 000 000 g/mol, preferably up to 2 000 000 g/mol.

Examples of cyclic ethers are 1,4-dioxane, tetrahydrofuran, and their derivatives like 2-methyl tetrahydrofuran.

Examples of acyclic acetals are 1,1-dimethoxymethane and 1,1-diethoxymethane. Examples of cyclic acetals are 1,3-dioxane, 1,3-dioxolane, and their derivatives such as methyl dioxolane.

Examples of acyclic orthocarboxylic acid esters are tri-C₁-C₄ alkoxy methane, in particular trimethoxymethane and triethoxymethane. Examples of suitable cyclic orthocarboxylic acid esters are 1,4-dimethyl-3,5,8-trioxabicyclo[2.2.2]octane and 4-ethyl-1-methyl-3,5,8-tri oxabicyclo[2.2.2]octane.

Examples of acyclic esters of carboxylic acids are ethyl and methyl formiate, ethyl and methyl acetate, ethyl and methyl proprionate, and ethyl and methyl butanoate, and esters of dicarboxylic acids like 1,3-dimethyl propanedioate. An example of a cyclic ester of carboxylic acids (lactones) is γ-butyrolactone.

Examples of cyclic and acyclic sulfones are ethyl methyl sulfone, dimethyl sulfone, and tetrahydrothiophene-S,S-dioxide (sulfolane).

Examples of cyclic and acyclic nitriles and dinitriles are adipodinitrile, acetonitrile, propionitrile, and butyronitrile.

The inventive electrolyte composition usually contains at least one conducting salt. The electrolyte composition functions as a medium that transfers ions participating in the electrochemical reaction taking place in an electrochemical cell. The conducting salt(s) present in the electrolyte are usually solvated in the aprotic organic solvent(s). The conducting salt is a lithium conducting salt. The conducting salt is preferably selected from the group consisting of
- Li[F₆₋ₓP(C_{y}F_{2y+1})ₓ], wherein x is an integer in the range from 0 to 6 and y is an integer in the range from 1 to 20;
   Li[B(R^{I})4], Li[B(R^{I})₂(OR^{II}O)] and Li[B(OR^{II}O)₂] wherein each R^{I} is independently from each other selected from F, Cl, Br, I, C₁-C₄ alkyl, C₂-C₄ alkenyl, C₂-C₄ alkynyl, OC₁-C₄ alkyl, OC₂-C₄ alkenyl, and OC₂-C₄ alkynyl wherein alkyl, alkenyl, and alkynyl may be substituted by one or more OR^{III}, wherein R^{III} is selected from C₁-C₆ alkyl, C₂-C₆ alkenyl, and C₂-C₆ alkynyl, and
   (OR^{II}O) is a bivalent group derived from a 1,2- or 1,3-diol, a 1,2- or 1,3-dicarboxlic acid or a 1,2- or 1,3-hydroxycarboxylic acid, wherein the bivalent group forms a 5- or 6-membered cycle via the both oxygen atoms with the central B-atom;
- LiClO₄; LiAsF₆; LiCF₃SO₃; Li₂SiF₆; LiSbF₆; LiAlCl₄, Li(N(SO₂F)₂), lithium tetrafluoro (oxalato) phosphate; lithium oxalate; and
- salts of the general formula Li[Z(CₙF₂ₙ₊₁SO₂)ₘ], where m and n are defined as follows:
   m = 1 when Z is selected from oxygen and sulfur,
   m = 2 when Z is selected from nitrogen and phosphorus,
   m = 3 when Z is selected from carbon and silicon, and
   n is an integer in the range from 1 to 20.

Suited 1,2- and 1,3-diols from which the bivalent group (OR^{II}O) is derived may be aliphatic or aromatic and may be selected, e.g., from 1,2-dihydroxybenzene, propane-1,2-diol, butane-1,2-diol, propane-1,3-diol, butan-1,3-diol, cyclohexyl-trans-1,2-diol and naphthalene-2,3-diol which are optionally are substituted by one or more F and/or by at least one straight or branched non fluorinated, partly fluorinated or fully fluorinated C₁-C₄ alkyl group. An example for such 1,2- or 1,3-diole is 1,1,2,2-tetra(trifluoromethyl)-1,2-ethane diol.

"Fully fluorinated C₁-C₄ alkyl group" means, that all H-atoms of the alkyl group are substituted by F.

Suited 1,2- or 1,3-dicarboxlic acids from which the bivalent group (OR^{II}O) is derived may be aliphatic or aromatic, for example oxalic acid, malonic acid (propane-1,3-dicarboxylic acid), phthalic acid or isophthalic acid, preferred is oxalic acid. The 1,2- or 1,3-dicarboxlic acid are optionally substituted by one or more F and/or by at least one straight or branched non fluorinated, partly fluorinated or fully fluorinated C₁-C₄ alkyl group.

Suited 1,2- or 1,3-hydroxycarboxylic acids from which the bivalent group (OR^{II}O) is derived may be aliphatic or aromatic, for example salicylic acid, tetrahydro salicylic acid, malic acid, and 2-hydroxy acetic acid, which are optionally substituted by one or more F and/or by at least one straight or branched non fluorinated, partly fluorinated or fully fluorinated C₁-C₄ alkyl group. An example for such 1,2- or 1,3-hydroxycarboxylic acids is 2,2-bis(trifluoromethyl)-2-hydroxy-acetic acid.

Examples of Li[B(R^{I})₄], Li[B(R^{I})₂(OR^{II}O)] and Li[B(OR^{II}O)₂] are LiBF₄, lithium difluoro oxalato borate and lithium dioxalato borate.

Preferably the at least one conducting salt (ii) is selected from Li[N(FSO₂)₂], Li[N(CF₃SO₂)₂], LiClO₄, LiPF₆, LiBF₄, and LiPF₃(CF₂CF₃)₃, more preferred the conducting salt (ii) is selected from LiPF₆ and LiBF₄, and the most preferred conducting salt (ii) is LiPF₆.

The at least one conducting salt is usually present at a minimum concentration of at least 0.1 mol/l, preferably the concentration of the at least one conducting salt is 0.5 to 2 mol/l based on the entire electrolyte composition.

The electrolyte composition according to the present invention may contain at least one further additive different from the compounds of formula (I) and formula (II). The further additive may be selected from polymers, SEI forming additives, flame retardants, overcharge protection additives, wetting agents, HF and/or H₂O scavenger, stabilizer for LiPF₆ salt, ionic solvation enhancer, corrosion inhibitors, gelling agents, and the like.
Examples for polymers used in electrolyte compositions are polyvinylidene fluoride, polyvinylidene-hexafluoropropylene copolymers, polyvinylidene-hexafluoropropylene-chlorotrifluoroethylene copolymers, Nafion, polyethylene oxide, polymethyl methacrylate, polyacrylonitrile, polypropylene, polystyrene, polybutadiene, polyethylene glycol, polyvinylpyrrolidone, polyaniline, polypyrrole and/or polythiophene. These polymers may be added to electrolyte compositions containing a solvent or solvent mixture in order to convert liquid electrolytes into quasi-solid or solid electrolytes and thus to improve solvent retention, especially during ageing.

Examples of flame retardants are organic phosphorous compounds like cyclophosphazenes, phosphoramides, alkyl and/or aryl tri-substituted phosphates, alkyl and/or aryl di- or tri-substituted phosphites, alkyl and/or aryl di- substituted phosphonates, alkyl and/or aryl tri-substituted phosphines, and fluorinated derivatives thereof.

Examples of HF and/or H₂O scavenger are optionally halogenated cyclic and acyclic silylamines.

Examples of overcharge protection additives are cyclohexylbenzene, o-terphenyl, p-terphenyl, and biphenyl and the like, preferred are cyclohexylbenzene and biphenyl.

Examples of SEI forming additives are vinylene carbonate and its derivatives such as vinylene carbonate and methylvinylene carbonate; fluorinated ethylene carbonate and its derivatives such as monofluoroethylene carbonate, cis- and trans-difluorocarbonate; propane sultone and its derivatives; ethylene sulfite and its derivatives; oxalate comprising compounds such as lithium oxalate, oxalato borates including dimethyl oxalate, lithium bis(oxalate) borate, lithium difluoro (oxalato) borate, and ammonium bis(oxalato) borate, and oxalato phosphates including lithium tetrafluoro (oxalato) phosphate; lithium fluorophosphates including LiPO₂F₂; and ionic compounds of formula (II) K⁺A- containing a cation K⁺ of formula (IIa) 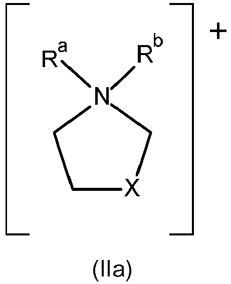 wherein
X is CH₂ or NR^{c},
R^{a} is selected from C₁ to C₆ alkyl,
R^{b} is selected from -(CH₂)ᵤ-SO₃-(CH₂)ᵥ-R^{d},
-SO₃- is -O-S(O)₂- or -S(O)₂-O-, preferably -SO₃- is -O-S(O)₂-,
u is an integer from 1 to 8, preferably u is 2, 3 or 4, wherein one or more CH₂ groups of the - (CH₂)ᵤ- alkylene chain which are not directly bound to the N-atom and/or the SO₃ group may be replaced by O and wherein two adjacent CH₂ groups of the -(CH₂)ᵤ- alkylene chain may be replaced by a C-C double bond, preferably the -(CH₂)ᵤ- alkylene chain is not substituted and u u is an integer from 1 to 8, preferably u is 2, 3 or 4,
v is an integer from 1 to 4, preferably v is 0,
R^{c} is selected from C₁ to C₆ alkyl,
R^{d} is selected from C₁-C₂₀ alkyl, C₂-C₂₀ alkenyl, C₂-C₂₀ alkynyl, C₆-C₁₂ aryl, and C₆-C₂₄ aralkyl, which may contain one or more F, and wherein one or more CH₂ groups of alkyl, alkenyl, alkynyl and aralkyl which are not directly bound to the SO₃ group may be replaced by O, preferably R^{b} is selected from C₁-C₆ alkyl, C₂-C₄ alkenyl, and C₂-C₄ alkynyl, which may contain one or more F, and wherein one or more CH₂ groups of alkyl, alkenyl, alkynyl and aralkyl which are not directly bound to the SO₃ group may be replaced by O, preferred examples of R^{b} include methyl, ethyl, trifluoromethyl, pentafluoroethyl, n-propyl, n-butyl, n-hexyl, ethenyl, ethynyl, allyl or prop-1-yn-yl,
and an anion A⁻ selected from bisoxalato borate, difluoro (oxalato) borate, [F_{z}B(CₘF₂ₘ₊₁)_{4-z}]⁻, [F_{y}P(CₘF₂ₘ₊₁)_{6-y}]⁻, (CₘF₂ₘ₊₁)₂P(O)O]⁻, [CₘF₂ₘ₊₁P(O)O₂]²⁻, [O-C(O)-CₘF₂ₘ₊₁]⁻, [O-S(O)₂-CₘF₂ₘ₊₁]⁻, [N(C(O)-CₘF₂ₘ₊₁)₂]⁻, [N(S(O)₂-CₘF₂ₘ₊₁)₂]⁻, [N(C(O)-CₘF₂ₘ₊₁)(S(O)₂-CₘF₂ₘ₊₁)]⁻, [N(C(O)-CₘF₂ₘ₊₁)(C(O)F)]⁻, [N(S(O)₂-CₘF₂ₘ₊₁)(S(O)₂F)]⁻, [N(S(O)₂F)₂]⁻, [C(C(O)-CₘF₂ₘ₊₁)₃]⁻, [C(S(O)₂-CₘF₂ₘ₊₁)₃]⁻, wherein m is an integer from 1 to 8, z is an integer from 1 to 4, and y is an integer from 1 to 6,

Preferred anions A⁻ are bisoxalato borate, difluoro (oxalato) borate, [F₃B(CF₃)]⁻, [F₃B(C₂F₅)]⁻, [PF_{6]}⁻, [F₃P(C₂F₅)₃]⁻, [F₃P(C₃F₇)₃]⁻, [F₃P(C₄F₉)₃]⁻, [F₄P(C₂F₅)₂]⁻, [F₄P(C₃F₇)₂]⁻, [F₄P(C₄F₉)₂]⁻, [F₅P(C₂F₅)]⁻, [F₅P(C₃F₇)]⁻ or [F₅P(C₄F₉)]⁻, [(C₂F₅)₂P(O)O]⁻, [(C₃F₇)₂P(O)O]⁻ or [(C₄F₉)₂P(O)O]⁻. [C₂F₅P(O)O_{2]}²⁻, [C₃F₇P(O)O_{2]}²⁻, [C₄F₉P(O)O_{2]}²⁻, [O-C(O)CF₃]⁻, [O-C(O)C₂F₅]⁻, [O-C(O)C₄F₉]⁻, [O-S(O)₂CF₃]⁻, [O-S(O)₂C₂F₅]⁻, [N(C(O)C₂F₅)₂]⁻, [N(C(O)(CF₃)₂]⁻, [N(S(O)₂CF₃)₂]⁻, [N(S(O)₂C₂F₅)₂]⁻, [N(S(O)₂C₃F₇)₂]⁻, [N(S(O)₂CF₃) (S(O)₂C₂F₅)]⁻, [N(S(O)₂C₄F₉)₂]⁻, [N(C(O)CF₃)(S(O)₂CF₃)]⁻, [N(C(O)C₂F₅)(S(O)₂CF₃)]⁻ or [N(C(O)CF₃)(S(O)₂-C₄F₉)]⁻, [N(C(O)CF₃)(C(O)F)]⁻, [N(C(O)C₂F₅)(C(O)F)]⁻, [N(C(O)C₃F₇)(C(O)F)]⁻, [N(S(O)₂CF₃)(S(O)₂F)]⁻, [N(S(O)₂C₂F₅)(S(O)₂F)]⁻, [N(S(O)₂C₄F₉)(S(O)₂F)]⁻, [C(C(O)CF₃)₃]⁻, [C(C(O)C₂F₅)₃]⁻ or [C(C(O)C₃F₇)₃]⁻, [C(S(O)₂CF₃)₃]⁻, [C(S(O)₂C₂F₅)₃]⁻, and [C(S(O)₂C₄F₉)₃]⁻.

More preferred the anion is selected from bisoxalato borate, difluoro (oxalato) borate, CF₃SO₃⁻, and [PF₃(C₂F₅)₃]⁻. Compounds of formula (II) and their preparation are described in WO 2013/026854 A1.

Preferred SEI-forming additives are oxalato borates, fluorinated ethylene carbonate and its derivatives, vinylene carbonate and its derivatives, and compounds of formula (II). More preferred are lithium bis(oxalato) borate (LiBOB), lithium difluoro(oxalato) borate (LidFOB), lithium fluorophosphates (e.g. LiPO₂F₂) vinylene carbonate, monofluoro ethylene carbonate, and compounds of formula (II), in particular monofluoro ethylene carbonate, and compounds of formula (II).

A compound added as additive may have more than one effect in the electrolyte composition and the device comprising the electrolyte composition. E.g. lithium oxalato borate may be added as additive enhancing the SEI formation but it may also be added as conducting salt.

In case one or more further additives are present, the total concentration of all further additives is at least 0.05 wt.-%, based on the total amount of the electrolyte composition, preferred the total concentration of the one or more further additives is 0.1 to 30 wt.-%, more preferred 0.5 to 10 wt.-%.

According to one embodiment of the present invention the electrolyte composition contains at least one compound of formula (I) and at least one SEI forming additive, all as described above or as described as being preferred.

In one embodiment of the present invention, the electrolyte composition contains:
(i) at least one compound of formula (I),
(ii) at least one organic aprotic solvent,
(iii) at least one conducting salt, and
(iv) optionally at least one further additive different from the compounds of formula (I).

The electrolyte composition preferably contains
(i) in total 0.005 to 10 wt.-% of compound(s) of formula (I), preferred 0.01 to 5 wt.-%, even more preferred 0.05 to 2 wt.-%,
(ii) in total 60 to 99.9 wt.-% of organic aprotic solvent(s),
(iii) at minimum 0.1 mol/l of at least one conducting salt, preferably 0.5 to 2 mol/l, and
(iv) zero to in total 30 wt.-% of further additive(s) different from the compounds of formula (I), preferably in total 0.1 to 30 wt.-%, even more preferred in total 0.5 to 10 wt.-%,
based on the total amount of the electrolyte composition.

The inventive electrolyte composition is preferably liquid at working conditions; more preferred it is liquid at 1 bar and 25 °C, even more preferred the electrolyte composition is liquid at 1 bar and -15 °C.

The water content of the inventive electrolyte composition is preferably below 100 ppm, based on the weight of the electrolyte composition, more preferred below 50 ppm, most preferred below 30 ppm. The water content may be determined by titration according to Karl Fischer, e.g. described in detail in DIN 51777 or ISO760: 1978.

The content of HF of the inventive electrolyte composition is preferably below 200 ppm, based on the weight of the electrolyte composition, more preferred below 100 ppm, most preferred below 60 ppm. The HF content may be determined by titration according to potentiometric or potentiographic titration method.

The electrolyte compositions of the invention are prepared by methods which are known to the person skilled in the field of the production of electrolytes, generally by dissolving a conductiing salt in the corresponding solvent mixture and adding the compound(s) of the formula (I) according to the invention and optionally additional additives, as described above.

The electrolyte compositions are used in electrochemical cells like secondary lithium batteries, double layer capacitors, and lithium ion capacitors, preferably the inventive electrolyte compositions are used in secondary lithium batteries and more preferred in lithium ion batteries.

Another object of the present invention is an electrochemical cell comprising the electrolyte composition as described above.

The general construction of such electrochemical devices is known and is familiar to the person skilled in this art - for batteries, for example, in Linden's Handbook of Batteries (ISBN 978-0-07-162421-3).

The inventive electrochemical cell may be a secondary lithium battery, a double layer capacitor, or a lithium ion capacitor. Preferably the electrochemical cell is a secondary lithium battery. The term "secondary lithium battery" as used herein means a secondary electrochemical cell, wherein the anode comprises lithium metal or lithium ions sometime during the charge/discharge of the cell. The anode may comprise lithium metal or a lithium metal alloy, a material occluding and releasing lithium ions, or other lithium containing compounds; e.g. the lithium battery may be a lithium ion battery, a lithium/sulphur battery, or a lithium/selenium sulphur battery.

In particular preferred the electrochemical device is a lithium ion battery, i.e. a secondary lithium ion electrochemical cell comprising a cathode comprising a cathode active material that can reversibly occlude and release lithium ions and an anode comprising an anode active material that can reversibly occlude and release lithium ions. The terms "secondary lithium ion electrochemical cell" and "(secondary) lithium ion battery" are used interchangeably within the present invention.

The at least one cathode active material preferably comprises a material capable of occluding and releasing lithium ions selected from lithium transition metal phosphates and lithium intercalating metal oxides. The lithium is usually intercalated in form of lithium ions.

Examples of lithium transition metal phosphates are LiFePO₄ and LiCoPO₄, examples of lithium intercalating metal oxides are LiCoO₂, LiNiO₂, mixed transition metal oxides with layer structure having the general formula Li_{(1+z)}[NiₐCo_{b}Mn_{c}]_{(1-z)}O₂₊ₑ wherein z is 0 to 0.3; a, b and c may be same or different and are independently 0 to 0.8 wherein a + b + c = 1; and -0.1 ≤ e ≤ 0.1, and manganese-containing spinels like LiMnO₄ and spinels of general formula Li₁₊ₜM₂₋ₜO_{4-d} wherein d is 0 to 0.4, t is 0 to 0.4 and M is Mn and at least one further metal selected from the group consisting of Co and Ni, and Li_{(1+g})[NiₕCoᵢAlⱼ]_{(1-g)}O₂₊ₖ. Typical values for g, h, I, j and k are: g = 0, h = 0.8 to 0.85, i = 0.15 to 0.20, j = 0.02 to 0.03 and k = 0.

The cathode may further comprise electrically conductive materials like electrically conductive carbon and usual components like binders. Compounds suited as electrically conductive materials and binders are known to the person skilled in the art. For example, the cathode may comprise carbon in a conductive polymorph, for example selected from graphite, carbon black, carbon nanotubes, graphene or mixtures of at least two of the aforementioned substances. In addition, the cathode may comprise one or more binders, for example one or more organic polymers like polyethylene, polyacrylonitrile, polybutadiene, polypropylene, polystyrene, polyacrylates, polyvinyl alcohol, polyisoprene and copolymers of at least two comonomers selected from ethylene, propylene, styrene, (meth)acrylonitrile and 1,3-butadiene, especially styrene-butadiene copolymers, and halogenated (co)polymers like polyvinlyidene chloride, polyvinly chloride, polyvinyl fluoride, polyvinylidene fluoride (PVdF), polytetrafluoroethylene, copolymers of tetrafluoroethylene and hexafluoropropylene, copolymers of tetrafluoroethylene and vinylidene fluoride and polyacrylnitrile.

The anode comprised within the lithium batteries of the present invention comprises an anode active material that can reversibly occlude and release lithium ions or is capable to form an alloy with lithium. In particular carbonaceous material that can reversibly occlude and release lithium ions can be used as anode active material. Carbonaceous materials suited are crystalline carbon such as a graphite material, more particularly, natural graphite, graphitized cokes, graphitized MCMB, and graphitized MPCF; amorphous carbon such as coke, mesocarbon microbeads (MCMB) fired below 1500°C, and mesophase pitch-based carbon fiber (MPCF); hard carbon and carbonic anode active material (thermally decomposed carbon, coke, graphite) such as a carbon composite, combusted organic polymer, and carbon fiber.

Further anode active materials are lithium metal, or materials containing an element capable of forming an alloy with lithium. Non-limiting examples of materials containing an element capable of forming an alloy with lithium include a metal, a semimetal, or an alloy thereof. It should be understood that the term "alloy" as used herein refers to both alloys of two or more metals as well as alloys of one or more metals together with one or more semimetals. If an alloy has metallic properties as a whole, the alloy may contain a nonmetal element. In the texture of the alloy, a solid solution, a eutectic (eutectic mixture), an intermetallic compound or two or more thereof coexist. Examples of such metal or semimetal elements include, without being limited to, titanium (Ti), tin (Sn), lead (Pb), aluminum, indium (In), zinc (Zn), antimony (Sb), bismuth (Bi), gallium (Ga), germanium (Ge), arsenic (As), silver (Ag), hafnium (Hf), zirconium (Zr) yttrium (Y), and silicon (Si). Metal and semimetal elements of Group 4 or 14 in the long-form periodic table of the elements are preferable, and especially preferable are titanium, silicon and tin, in particular silicon. Examples of tin alloys include ones having, as a second constituent element other than tin, one or more elements selected from the group consisting of silicon, magnesium (Mg), nickel, copper, iron, cobalt, manganese, zinc, indium, silver, titanium (Ti), germanium, bismuth, antimony and chromium (Cr). Examples of silicon alloys include ones having, as a second constituent element other than silicon, one or more elements selected from the group consisting of tin, magnesium, nickel, copper, iron, cobalt, manganese, zinc, indium, silver, titanium, germanium, bismuth, antimony and chromium.

A further possible anode active material are silicon based materials. Silicon based materials include silicon itself, e.g. amorphous and crystalline silicon, silicon containing compounds like SiOₓ with 0 < x < 1.5 and Si alloys, and compositions containing silicon and/or silicon containing compounds, e.g. silicon/graphite composites. The silicon may be used in different forms, e.g. in the form of nanowires, nanotubes, nanoparticles, films, nanoporous silicon or silicon nanotubes. The silicon may be deposited on a current collector. The current collector may be a metal wire, a metal grid, a metal web, a metal sheet, a metal foil or a metal plate. Preferred the current collector is a metal foil, e.g. a copper foil. Thin films of silicon may be deposited on metal foils by any technique known to the person skilled in the art, e.g. by sputtering techniques. One possibility of preparing Si thin film electrodes are described in R. Elazari et al.; Electrochem. Comm. 2012, 14, 21-24.

Other possible anode active materials are lithium ion intercalating oxides of Ti.

Preferably the anode active material is selected from carbonaceous material that can reversibly occlude and release lithium ions, particularly preferred the carbonaceous material that can reversibly occlude and release lithium ions is selected from crystalline carbon, hard carbon and amorphous carbon, in particular preferred is graphite. In another preferred embodiment the anode active is selected from silicon that can reversibly occlude and release lithium ions, preferably the anode comprises a thin film of silicon or a silicon/carbon mixture. In a further preferred embodiment the anode active is selected from lithium ion intercalating oxides of Ti.

The anode and cathode may be made by preparing an electrode slurry composition by dispersing the electrode active material, a binder, optionally a conductive material and a thickener, if desired, in a solvent and coating the slurry composition onto a current collector. The current collector may be a metal wire, a metal grid, a metal web, a metal sheet, a metal foil or a metal plate. Preferred the current collector is a metal foil, e.g. a copper foil or aluminum foil.

The inventive lithium batteries may contain further constituents customary per se, for example separators, housings, cable connections etc. The housing may be of any shape, for example cuboidal or in the shape of a cylinder, the shape of a prism or the housing used is a metal-plastic composite film processed as a pouch. Suited separators are for example glass fiber separators and polymer-based separators like polyolefin separators.
Several inventive lithium batteries may be combined with one another, for example in series connection or in parallel connection. Series connection is preferred. The present invention further provides for the use of inventive lithium ion batteries as described above in devices, especially in mobile devices. Examples of mobile devices are vehicles, for example automobiles, bicycles, aircraft, or water vehicles such as boats or ships. Other examples of mobile devices are those which are portable, for example computers, especially laptops, telephones or electrical power tools, for example from the construction sector, especially drills, battery-driven screwdrivers or battery-driven tackers. But the inventive lithium ion batteries can also be used for stationary energy stores.

Even without further statements, it is assumed that a skilled person is able to utilize the above description in its widest extent. Consequently, the preferred embodiments and examples are to be interpreted merely as a descriptive enclosure which in no way has any limiting effect at all.

The invention is illustrated by the examples which follow, which do not, however, restrict the invention.

### I. Preparation of cyclic dinitriles of formula (I)

### 1.1 5,6-dimethoxypyrazin-2,3-dicarbonitril (Compound 1.1)

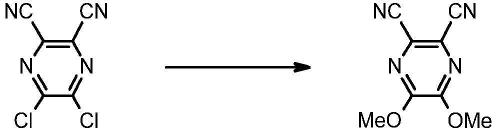

A mixture of 5,6-dichloro-2,3-dicyanopyrazine (10.54 g, 52.97 mmol) and methanol (MeOH) (270 ml) was warmed to 50°C and triethylamine (10.93 g, 108 mmol) was added dropwise.

The mixture was heated to reflux and stirred for 20 hours. After cooling to room temperature (RT) the mixture was concentrated on the rotary evaporator, then water (400 ml) and CH₂Cl₂ (400 ml) were added and the phases separated. The aqueous phase was extracted with CH₂Cl₂ and the combined organic phases washed with water, dried over Na₂SO₄ and the solvent evaporated. The crude product (9.05 g) was dissolved in warm tetrahydrofurane (THF) (200 ml) and ice-water (200 ml) was added. The precipitated solid was collected by filtration, washed with cyclohexane and dried to provide the desired product as green solid (5.94 g).

### 1.2 5,6-dibenzyloxypyrazin-2,3-dicarbonitril (Compound 1.2)

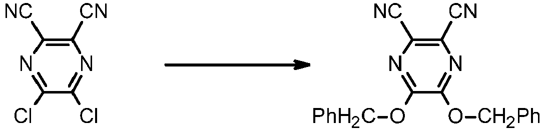

To a mixture of 5,6-dichloro-2,3-dicyanopyrazine (10.16 g, 51.06 mmol) in THF (270 ml) triethylamine (10.59 g, 104.65 mmol) was added at RT. Then benzylalcohol (13.8 g, 127.6 mmol) was added dropwise at RT. AFterwards the mixture was heated to reflux, stirred for 18 hours and cooled to RT. The solids were filtered off and to the filtrate ice-water (1000 ml) was added. The precipitated solid was collected by filtration, washed with cyclohexane and dried. The crude product (7.08 g) was recrystallized (acetone/n-hexane) to give the desired product as yellow-green solid (5.02 g).

### 1.3 5,6-diphenylpyrazin-2,3-dicarbonitril (Compound 1.3)

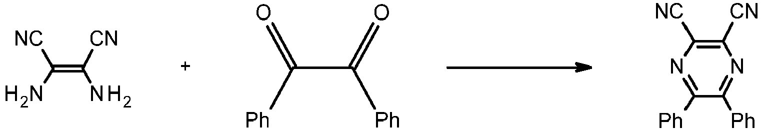

To a mixture of diaminomaleonitrile (2.16g, 20 mmol) and H₂SO₄ (1 ml) in MeOH (50 ml) was added a solution of 1,2-diphenylethane-1,2-dione (4.30 g, 20 mmol) in MeOH (150 ml) at 45°C. A precipitate started to form. The mixture was stirred 2 hours at 45°C and 1.5 hours at reflux. After cooling to RT the precipitate was filtered, washed with cyclohexane and dried to give the desired product (5.27 g) as beige solid.

### 1.4 5,6-bis(phenylsulfanyl)pyrazin-2,3-dicarbonitril (Compound 1.4)

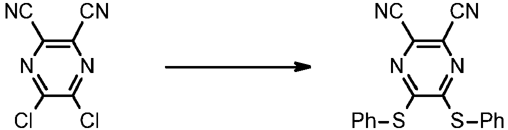

To a mixture of 5,6-dichloro-2,3-dicyanopyrazine (10.00 g, 50.25 mmol) in THF (270 ml) triethylamine (10.42 g, 102.97 mmol) was added at RT. Then thiophenol (12.18 g, 110.55 mmol) was added dropwise during which the temperature raised from RT to 41 °C and a white precipitate was formed. The mixture was stirred at reflux overnight then cooled to RT and the solids were filtered off. The filtrate was concentrated and CH₂Cl₂ (200 ml) and aq. NaHCO₃ (200 ml) were added to the residue and the phases separated. The organic phase was washed with aq. NaHCO₃ (200 ml) and water (2x200 ml), dried over Na₂SO₄ and the solvent evaporated. The crude product (14.3 g) was recrystallized from isopropanol to give the desired product as brown solid (9.37 g).

### 1.5 5,6-bis(2,2,2-trifluorethoxy)pyrazin-2,3-dicarbonitril (Compound 1.5)

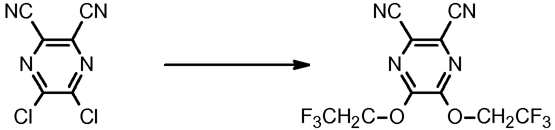

To a mixture of NaH (4.2 g, 60% in mineral oil, 0.11 mmol) in THF (50 ml) 2,2,2-trifluoroethanol (10.75 g, 0.11 mmol) in THF (50 ml) was added dropwise at 0°C over 30 min. After stirring for 2 hours, a solution of 5,6-dichloro-2,3-dicyanopyrazine (10.2 g, 50 mmol) in THF (50 ml) was added over 12 min at 0°C. The reaction mixture was stirred 1 hour at 0°C then warmed to RT and stirred overnight. The mixture was quenched with aq. NH₄Cl (100 ml) and extracted with CH₂Cl₂ (1x100 ml and 2x50 ml), the combined organic phases dried over Na₂SO₄ and the solvent evaporated. The crude product was recrystallized from isopropanol and dried to give the desired product (7.14 g) as yellow-green solid.

### 1.6 5,6-bis(benzylsulfanyl)pyrazin-2,3-dicarbonitril (Compound 1.6)

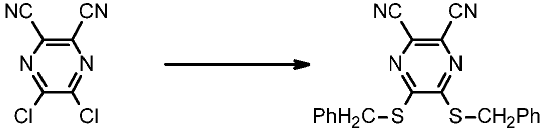

To a mixture of 5,6-dichloro-2,3-dicyanopyrazine (10.00 g, 50.25 mmol) in THF (270 ml) triethylamine (10.42 g, 102.97 mmol) was added at RT. Then benzyl mercaptan (13.73 g, 110.54 mmol) was added dropwise, during which the temperature raised from RT to 50°C. The mixture was stirred at RT for 5 hours then cooled to 0°C and the solids were filtered off. The filtrate was concentrated and CH₂Cl₂ (200 ml) and aq. NaHCO₃ (200 ml) were added to the residue and the phases separated. The organic phase was washed with aq. NaHCO₃ (200 ml) and water (200 ml), dried over Na₂SO₄ and the solvent evaporated. The crude product (19 g) was recrystallized from isopropanol to give the desired product as violet solid (12.8 g).

### II. Electrolyte compositions

Electrolyte compositions were prepared from ethylene carbonate (EC), diethyl carbonate (DEC), LiPF₆, adiponitrile, 1,4,5,6-tetrahydro-5,6-dioxo-2,3-pyrazinedicarbonitrile and compounds 1.1 to 1.4. The compositions are indicated in Table 1, "wt.-%" are based on the total weight of the electrolyte composition.

### III. Electrochemical cells.

Commercially available wound pouch dry cells (Lithium Cobalt Oxide vs Graphite) were dried at 70°C *in vacuo* for 24h then filled with 700 µl electrolyte under Argon atmosphere. After 5h rest at room temperature the cells were evacuated and sealed. The cells were then cycled between 2.75 and 4.4V for 5 cycles, charged to 4.4V and stored at 85°C for 24h.

The volume change of the cells was measured before and after 85°C storage by using Archimedes' principle. This method is known to those skilled in the art. The results of the experiments are shown in Table 1.

The amount of Co dissolved from the cathode in electrolyte and migrated to the graphite electrode was determined by ICP-OES (inductively coupled plasma optical emission spectrometry) after storage at 85°C for 24h. The lower the value the better. All values are normalized to comparative example 2 with comparative example 2 being assigned a value of 100. The results of the experiments are shown in Table 1.

**Table 1**

| | Electrolyte composition | Delta volume (%) | Co dissolution |
|---|---|---|---|
| Example 1 (comparative) | EC:EMC 3:7 w:w 1 MLiPF₆ + 0.5 wt.-% 1,4,5,6-tetrahydro-5,6-dioxo-2,3-pyrazinedicarbonitrile | 3.7 | 84 |
| Example 2 (comparative) | EC:EMC 3:7 w:w 1 MLiPF₆ | 2.5 | 100 |
| Example 3 (comparative) | EC:EMC 3:7 w:w 1 MLiPF₆ + 0.34 wt.-% adiponitrile | 4.6 | 116 |
| Example 4 (inventive) | EC:EMC 3:7 w:w 1 MLiPF₆ + 0.59 wt.-% compound (I.1) | 2.7 | 135 |
| Example 5 (inventive) | EC:EMC 3:7 w:w 1 MLiPF₆ + 1.05 wt.-% compound (I.2) | 0.9 | 63 |
| Example 6 (inventive) | EC:EMC 3:7 w:w 1 MLiPF₆ + 0.5 wt.-% compound (I.3) | 3.5 | 116 |
| Example 7 (inventive) | EC:EMC 3:7 w:w 1 MLiPF₆ + 1.1 wt.-% compound (I.4) | 2.6 | 69 |

All inventive electrolyte compositions containing a cyclic dinitrile of formula (I) show at least lower Co dissolution or lower change of volume after 24h storage at 85 °C than electrolyte compositions containing a dinitrile already known as electrolyte additive.

## Claims

1. An electrolyte composition containing at least one conducting salt selected from lithium salts and at least one compound of formula (I) 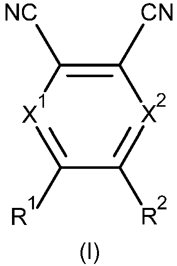 wherein
X¹ and X² are N;
R¹ and R² are selected independently from each other from R⁴, OR⁴, SR⁴, S(O)R⁴, S(O)₂R⁴, OS(O)₂R⁴, S(O)₂OR⁴, OSiR⁸₃, and NR⁵R⁶,
R⁴ is selected from C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F, CN, C(O)OR⁷, OC(O)R⁷, OR⁷, and SR⁷; R⁵ and R⁶ are selected independently from each other from H, C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
or R⁵ and R⁶ may be combined to form together with the C- or N-atom a 5- to 7-membered heterocycle which may be substituted by one or more substituents selected from F, CN, C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
R⁷ is selected from C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl which may be substituted by one or more substituents selected from F and CN; and
R⁸ is independently selected from R⁴ and OR⁴.

2. The electrolyte composition according to claim 1, wherein R¹ and R² are selected independently from each other from OR⁴, SR⁴, S(O)₂R⁴, OS(O)₂R⁴, S(O)₂OR⁴, OSiR⁸₃, and NR⁵R⁶.

3. The electrolyte composition according to claim 1 or 2, wherein R¹ and R² are independently from each other selected from R⁴.

4. The electrolyte composition according to any of claims 1 to 3, wherein R¹ and R² are equal.

5. The electrolyte composition according to any of claims 1 to 4, wherein the compound of formula (I) is selected from the compounds of formula (I) wherein
X¹ and X² are N,
R¹ and R² are selected independently from each other from R⁴, OR⁴, and SR⁴, and
R⁴ is selected from C₁ to C₁₂ alkyl, C₅ to C₁₂ (hetero)aryl, and C₆ to C₂₄ (hetero)aralkyl wherein alkyl, (hetero)aryl, and hetero(aralkyl) may be substituted by one or more substituents selected from F and CN.

6. The electrolyte composition according to any of claims 1 to 5, wherein the compound of formula (I) is selected from the compounds of formula (I) wherein
(1.1) X¹ and X² are N and R¹ and R² are O-CH₂-phenyl or O-CH₂-CF₃,
(1.2) X¹ and X² are N and R¹ and R² are S-phenyl or S-CH₂-Phenyl,
(1.3) X¹ and X² are N and R¹ and R² are phenyl or ethynyl, or
(1.4) X¹ and X² are N and R¹ and R² are OCH₃.

7. The electrolyte composition according to any of claims 1 to 6, wherein the electrolyte composition contains in total 0.005 to 10 wt.-% of the at least one compound of formula (I), based on the total weight of the electrolyte composition.

8. The electrolyte composition according to any of claims 1 to 7, wherein the electrolyte composition contains at least one aprotic organic solvent, at least one lithium conducting salt and optionally at least one further additive different from the compounds of formula (I).

9. Use of a compound of formula (I) 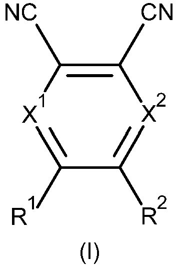 wherein
X¹ and X² are N;
R¹ and R² are selected independently from each other from R⁴, OR⁴, SR⁴, S(O)R⁴, S(O)₂R⁴, OS(O)₂R⁴, S(O)₂OR⁴, OSiR⁸₃, and NR⁵R⁶
R⁴ is selected from C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F, CN, C(O)OR⁷, OC(O)R⁷, OR⁷, and SR⁷; R⁵ and R⁶ are selected independently from each other from H, C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
or R⁵ and R⁶ may be combined to form together with the C- or N-atom a 5- to 7-membered heterocycle which may be substituted by one or more substituents selected from F, CN, C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl, wherein alkyl, (hetero)cycloalkyl, alkenyl, alkynyl, (hetero)aryl, and (hetero)aralkyl may be substituted by one or more substituents selected from F and CN;
R⁷ is selected from C₁ to C₁₂ alkyl, C₃-C₆ (hetero)cycloalkyl, C₂ to C₁₂ alkenyl, C₂ to C₁₂ alkynyl, C₅ to C₁₂ (hetero)aryl, and C₆ to C₂₄ (hetero)aralkyl which may be substituted by one or more substituents selected from F and CN; and
R⁸ is independently selected from R⁴ and OR⁴;
in electrolyte compositions of electrochemical cells.

10. An electrochemical cell comprising the electrolyte composition according to any of claims 1 to 8.

11. The electrochemical cell according to claim 10 wherein the electrochemical cell is a secondary lithium battery.

## Patentansprüche

1. Elektrolytzusammensetzung, enthaltend mindestens ein Leitsalz, das aus Lithiumsalzen ausgewählt ist, und mindestens eine Verbindung der Formel (I) 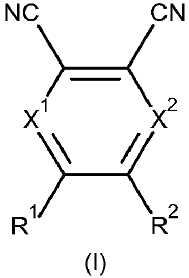 worin
X¹ und X² für N stehen;
R¹ und R² unabhängig voneinander aus R⁴, OR⁴, SR⁴, S(O)R⁴, S(O)₂R₄, OS(O)₂R⁴, S(O)₂OR⁴, OSiR⁸₃ und NR⁵R⁶ ausgewählt sind,
R⁴ aus C₁- bis C₁₂-Alkyl, C₃-C₆-(Hetero)cycloalkyl, C₂- bis C₁₂-Alkenyl, C₂- bis C₁₂-Alkinyl, C₅- bis C₁₂- (Hetero) aryl und C₆- bis C₂₄-(Hetero)aralkyl ausgewählt ist, wobei Alkyl, (Hetero)cycloalkyl, Alkenyl, Alkinyl, (Hetero)aryl und (Hetero)aralkyl durch einen oder mehrere Substituenten, die aus F, CN, C(O)OR⁷, OC(O)R⁷, OR⁷ und SR⁷ ausgewählt sind, substituiert sein können;
R⁵ und R⁶ unabhängig voneinander aus H, C₁- bis C₁₂-Alkyl, C₃-C₆-(Hetero)cycloalkyl, C₂- bis C₁₂-Alkenyl, C₂- bis C₁₂-Alkinyl, C₅- bis C₁₂-(Hetero)aryl und C₆- bis C₂₄-(Hetero)aralkyl ausgewählt sind, wobei Alkyl, (Hetero)cycloalkyl, Alkenyl, Alkinyl, (Hetero)aryl und (Hetero)aralkyl durch einen oder mehrere Substituenten, die aus F und CN ausgewählt sind, substituiert sein können;
oder R⁵ und R⁶ zusammen gemeinsam mit dem C- oder N-Atom einen 5- bis 7-gliedrigen Heterocyclus bilden können, der durch einen oder mehrere Substituenten, die aus F, CN, C₁- bis C₁₂-Alkyl, C₃-C₆-(Hetero)cycloalkyl, C₂- bis C₁₂-Alkenyl, C₂- bis C₁₂-Alkinyl, C₅- bis C₁₂- (Hetero) aryl und C₆- bis C₂₄-(Hetero)aralkyl ausgewählt sind, substituiert sein kann, wobei Alkyl, (Hetero)cycloalkyl, Alkenyl, Alkinyl, (Hetero)aryl und (Hetero)aralkyl durch einen oder mehrere Substituenten, die aus F und CN ausgewählt sind, substituiert sein können;
R⁷ aus C₁- bis C₁₂-Alkyl, C₃-C₆-(Hetero)cycloalkyl, C₂- bis C₁₂-Alkenyl, C₂- bis C₁₂-Alkinyl, C₅- bis C₁₂-(Hetero)aryl und C₆- bis C₂₄-(Hetero)aralkyl, die durch einen oder mehrere Substituenten, die aus F und CN ausgewählt sind, substituiert sein können, ausgewählt ist; und
R⁸ unabhängig aus R⁴ und OR⁴ ausgewählt ist.

2. Elektrolytzusammensetzung nach Anspruch 1, wobei R¹ und R² unabhängig voneinander aus OR⁴, SR⁴, S(O)₂R⁴, OS(O)₂R⁴, S(O)₂OR⁴, OSiR⁸₃ und NR⁵R⁶ ausgewählt sind.

3. Elektrolytzusammensetzung nach Anspruch 1 oder 2, wobei R¹ und R² unabhängig voneinander aus R⁴ ausgewählt sind.

4. Elektrolytzusammensetzung nach einem der Ansprüche 1 bis 3, wobei R¹ und R² gleich sind.

5. Elektrolytzusammensetzung nach einem der Ansprüche 1 bis 4, wobei die Verbindung der Formel (I) aus den Verbindungen der Formel (I), worin X¹ und X² für N stehen,
R¹ und R² unabhängig voneinander aus R⁴, OR⁴ und SR⁴ ausgewählt sind und
R⁴ aus C₁- bis C₁₂-Alkyl, C₅- bis C₁₂-(Hetero)aryl und C₆- bis C₂₄-(Hetero)aralkyl ausgewählt ist, wobei Alkyl, (Hetero)aryl und (Hetero)aralkyl durch einen oder mehrere Substituenten, die aus F und CN ausgewählt sind, substituiert sein können, ausgewählt ist.

6. Elektrolytzusammensetzung nach einem der Ansprüche 1 bis 5, wobei die Verbindung der Formel (I) aus den Verbindungen der Formel (I), worin (I.1)
X¹ und X² für N stehen und R¹ und R² für O-CH₂-Phenyl oder O-CH₂-CF₃ stehen,
(I.2) X¹ und X² für N stehen und R¹ und R² für S-Phenyl oder S-CH₂-Phenyl stehen,
(I.3) X¹ und X² für N stehen und R¹ und R² für Phenyl oder Ethinyl stehen oder
(I.4) X¹ und X² für N stehen und R¹ und R² für OCH₃ stehen,
ausgewählt ist.

7. Elektrolytzusammensetzung nach einem der Ansprüche 1 bis 6, wobei die Elektrolytzusammensetzung insgesamt 0,005 bis 10 Gew.-% der mindestens einen Verbindung der Formel (I), bezogen auf das Gesamtgewicht der Elektrolytzusammensetzung, enthält.

8. Elektrolytzusammensetzung nach einem der Ansprüche 1 bis 7, wobei die Elektrolytzusammensetzung mindestens ein aprotisches organisches Lösungsmittel, mindestens ein Lithium-Leitsalz und gegebenenfalls mindestens ein weiteres Additiv, das von den Verbindungen der Formel (I) verschieden ist, enthält.

9. Verwendung einer Verbindung der Formel (I) 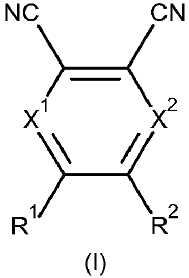 worin
X¹ und X² für N stehen;
R¹ und R² unabhängig voneinander aus R⁴, OR⁴, SR⁴, S(O)R⁴, S(O)₂R⁴, OS(O)₂R⁴, S(O)₂OR⁴, OSiR⁸₃ und NR⁵R⁶ ausgewählt sind,
R⁴ aus C₁- bis C₁₂-Alkyl, C₃-C₆-(Hetero)cycloalkyl, C₂- bis C₁₂-Alkenyl, C₂- bis C₁₂-Alkinyl, C₅- bis C₁₂- (Hetero) aryl und C₆- bis C₂₄-(Hetero)aralkyl ausgewählt ist, wobei Alkyl, (Hetero)cycloalkyl, Alkenyl, Alkinyl, (Hetero)aryl und (Hetero)aralkyl durch einen oder mehrere Substituenten, die aus F, CN, C(O)OR⁷, OC(O)R⁷, OR⁷ und SR⁷ ausgewählt sind, substituiert sein können;
R⁵ und R⁶ unabhängig voneinander aus H, C₁- bis C₁₂-Alkyl, C₃-C₆-(Hetero) cycloalkyl, C₂- bis C₁₂-Alkenyl, C₂- bis C₁₂-Alkinyl, C₅- bis C₁₂-(Hetero)aryl und C₆- bis C₂₄-(Hetero)aralkyl ausgewählt sind, wobei Alkyl, (Hetero)cycloalkyl, Alkenyl, Alkinyl, (Hetero)aryl und (Hetero)aralkyl durch einen oder mehrere Substituenten, die aus F und CN ausgewählt sind, substituiert sein können; oder R⁵ und R⁶ zusammen gemeinsam mit dem C- oder N-Atom einen 5- bis 7-gliedrigen Heterocyclus bilden, der durch einen oder mehrere Substituenten, die aus F, CN, C₁- bis C₁₂-Alkyl, C₃-C₆-(Hetero)cycloalkyl, C₂- bis C₁₂-Alkenyl, C₂- bis C₁₂-Alkinyl, C₅- bis C₁₂- (Hetero) aryl und C₆- bis C₂₄-(Hetero)aralkyl ausgewählt sind, substituiert sein kann, wobei Alkyl, (Hetero)cycloalkyl, Alkenyl, Alkinyl, (Hetero)aryl und (Hetero)aralkyl durch einen oder mehrere Substituenten, die aus F und CN ausgewählt sind, substituiert sein können; R⁷ aus C₁- bis C₁₂-Alkyl, C₃-C₆-(Hetero)cycloalkyl, C₂- bis C₁₂-Alkenyl, C₂- bis C₁₂-Alkinyl, C₅- bis C₁₂-(Hetero)aryl und C₆- bis C₂₄-(Hetero)aralkyl, die durch einen oder mehrere Substituenten, die aus F und CN ausgewählt sind, substituiert sein können, ausgewählt ist; und
R⁸ unabhängig aus R⁴ und OR⁴ ausgewählt ist;
in Elektrolytzusammensetzungen elektrochemischer Zellen.

10. Elektrochemische Zelle, umfassend die Elektrolytzusammensetzung nach einem der Ansprüche 1 bis 8.

11. Elektrochemische Zelle nach Anspruch 10, wobei es sich bei der elektrochemischen Zelle um eine sekundäre Lithiumbatterie handelt.

## Revendications

1. Composition d'électrolyte contenant au moins un sel conducteur choisi parmi les sels de lithium et au moins un composé de formule (I) 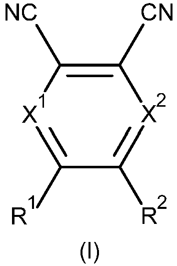 dans laquelle
chacun des chaînons X¹ et X² représente N ;
chacun des radicaux R¹ et R² est choisi indépendamment de l'autre parmi R⁴, OR⁴, SR⁴, S(O)R⁴, S(O)₂R⁴, OS(O)₂R⁴, S(O)₂OR⁴, OSiR⁸₃ et NR⁵R⁶,
R⁴ est choisi parmi les groupements alkyle en C₁ à C₁₂, (hétéro) cycloalkyle en C₃-C₆, alcényle en C₂ à C₁₂, alcynyle en C₂ à C₁₂, (hétéro) aryle en C₅ à C₁₂, et (hétéro) arylalkyle en C₆ à C₂₄, dans lequel les groupements alkyle, (hétéro)cycloalkyle, alcényle, alcynyle, (hétéro)aryle et (hétéro)arylalkyle peuvent être substitués par un ou plusieurs substituants choisis parmi F, CN, C(O)OR⁷, OC(O)R⁷, OR⁷ et SR⁷ ;
chacun des radicaux R⁵ et R⁶ est choisi indépendamment de l'autre parmi H, les groupements alkyle en C₁ à C₁₂, (hétéro) cycloalkyle en C₃-C₆, alcényle en C₂ à C₁₂, alcynyle en C₂ à C₁₂, (hétéro) aryle en C₅ à C₁₂, et (hétéro) arylalkyle en C₆ à C₂₄, dans lequel les groupements alkyle, (hétéro)cycloalkyle, alcényle, alcynyle, (hétéro)aryle et (hétéro)arylalkyle peuvent être substitués par un ou plusieurs substituants choisis parmi F et CN ;
ou R⁵ et R⁶ peuvent être associés pour former conjointement avec l'atome C ou N un hétérocycle comportant 5 à 7 chaînons qui peut être substitué par un ou plusieurs substituants choisis parmi F, CN, les groupements alkyle en C₁ à C₁₂, (hétéro) cycloalkyle en C₃-C₆, alcényle en C₂ à C₁₂, alcynyle en C₂ à C₁₂, (hétéro) aryle en C₅ à C₁₂, et (hétéro) arylalkyle en C₆ à C₂₄, dans lequel les groupements alkyle, (hétéro)cycloalkyle, alcényle, alcynyle, (hétéro)aryle et (hétéro)arylalkyle peuvent être substitués par un ou plusieurs substituants choisis parmi F et CN ;
R⁷ est choisi parmi les groupements alkyle en C₁ à C₁₂, (hétéro) cycloalkyle en C₃-C₆, alcényle en C₂ à C₁₂, alcynyle en C₂ à C₁₂, (hétéro) aryle en C₅ à C₁₂, et (hétéro) arylalkyle en C₆ à C₂₄ qui peuvent être substitués par un ou plusieurs substituants choisis parmi F et CN ; et
R⁸ est indépendamment choisi parmi R⁴ et OR⁴.

2. Composition d'électrolyte selon la revendication 1, dans lequel chacun des radicaux R¹ et R² est choisi indépendamment de l'autre parmi OR⁴, SR⁴, S(O)²R⁴, OS(O)₂R⁴, S(O)₂OR⁴, OSiR⁸₃ et NR⁵R⁶.

3. Composition d'électrolyte selon la revendication 1 ou 2, dans laquelle chacun des radicaux R¹ et R² est choisi indépendamment de l'autre parmi R⁴.

4. Composition d'électrolyte selon l'une quelconque des revendications 1 à 3, dans laquelle R¹ et R² sont égaux.

5. Composition d'électrolyte selon l'une quelconque des revendications 1 à 4, dans laquelle le composé de formule (I) est choisi parmi les composés de formule (I) dans laquelle
chacun des chaînons X¹ et X² représente N,
chacun des radicaux R¹ et R² est choisi indépendamment des autres parmi R⁴, OR⁴ et SR⁴, et
R⁴ est choisi parmi les groupements alkyle en C₁ à C₁₂, (hétéro)aryle en C₅ à C₁₂, et (hétéro) arylalkyle en C₆ à C₂₄ dans lequel les groupements alkyle, (hétéro)aryle et hétéro(arylalkyle) peuvent être substitués par un ou plusieurs substituants choisis parmi F et CN.

6. Composition d'électrolyte selon l'une quelconque des revendications 1 à 5, dans laquelle le composé de formule (I) est choisi parmi les composés de formule (I) dans laquelle
(I.1) chacun des chaînons X¹ et X² représente N et chacun des radicaux R¹ et R² représente O-CH₂-phényle ou O-CH₂-CF₃,
(I.2) chacun des chaînons X¹ et X² représente N et chacun des radicaux R¹ et R² représente S-phényle ou S-CH₂-Phényle,
(I.3) chacun des chaînons X¹ et X² représente N et chacun des radicaux R¹ et R² représente un groupement phényle ou éthynyle, ou
(I.4) chacun des chaînons X¹ et X² représente N et chacun des radicaux R¹ et R² représente OCH₃.

7. Composition d'électrolyte selon l'une quelconque des revendications 1 à 6, dans laquelle la composition d'électrolyte contient au total 0,005 à 10 % en masse de l'au moins un composé de formule (I), par rapport à la masse totale de la composition d'électrolyte.

8. Composition d'électrolyte selon l'une quelconque des revendications 1 à 7, dans laquelle la composition d'électrolyte contient au moins un solvant organique aprotique, au moins un sel conducteur à base de lithium et éventuellement au moins un adjuvant supplémentaire différent des composés de formule (I).

9. Utilisation d'un composé de formule (I) 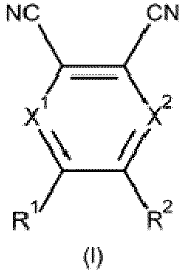 dans laquelle
chacun des chaînons X¹ et X² représente N ;
chacun des radicaux R¹ et R² est choisi indépendamment des autres parmi R⁴, OR⁴, SR⁴, S(O)R¹, S(O)₂R⁴, OS(O)₂R⁴, S(O)₂OR⁴, OSiR⁸₃ et NR⁵R⁶
R⁴ est choisi parmi les groupements alkyle en C₁ à C₁₂, (hétéro) cycloalkyle en C₃-C₆, alcényle en C₂ à C₁₂, alcynyle en C₂ à C₁₂, (hétéro) aryle en C₅ à C₁₂, et (hétéro) arylalkyle en C₆ à C₂₄, dans lequel les groupements alkyle, (hétéro)cycloalkyle, alcényle, alcynyle, (hétéro)aryle et (hétéro)arylalkyle peuvent être substitués par un ou plusieurs substituants choisis parmi F, CN, C(O)OR⁷, OC(O)R⁷, OR⁷ et SR⁷ ;
chacun des radicaux R⁵ et R⁶ est choisi indépendamment de l'autre parmi H, les groupements alkyle en C₁ à C₁₂, (hétéro) cycloalkyle en C₃-C₆, alcényle en C₂ à C₁₂, alcynyle en C₂ à C₁₂, (hétéro) aryle en C₅ à C₁₂, et
(hétéro) arylalkyle en C₆ à C₂₄, dans lequel les groupements alkyle, (hétéro)cycloalkyle, alcényle, alcynyle, (hétéro)aryle et (hétéro)arylalkyle peuvent être substitués par un ou plusieurs substituants choisis parmi F et CN ;
ou R⁵ et R⁶ peuvent être associés pour former conjointement avec l'atome C ou N un hétérocycle comportant 5 à 7 chaînons qui peut être substitué par un ou plusieurs substituants choisis parmi F, CN, les groupements alkyle en C₁ à C₁₂, (hétéro) cycloalkyle en C₃-C₆, alcényle en C₂ à C₁₂, alcynyle en C₂ à C₁₂, (hétéro)aryle en C₅ à C₁₂, et (hétéro)arylalkyle en C₆ à C₂₄, dans lequel les groupements alkyle, (hétéro)cycloalkyle, alcényle, alcynyle, (hétéro)aryle et (hétéro)arylalkyle peuvent être substitués par un ou plusieurs substituants choisis parmi F et CN ;
R⁷ est choisi parmi les groupements alkyle en C₁ à C₁₂, (hétéro) cycloalkyle en C₃-C₆, alcényle en C₂ à C₁₂, alcynyle en C₂ à C₁₂, (hétéro) aryle en C₅ à C₁₂, et (hétéro) arylalkyle en C₆ à C₂₄ qui peuvent être substitués par un ou plusieurs substituants choisis parmi F et CN ; et
R⁸ est indépendamment choisi parmi R⁴ et OR⁴ ;
dans les compositions d'électrolyte de cellules électrochimiques.

10. Cellule électrochimique comprenant la composition d'électrolyte selon l'une quelconque des revendications 1 à 8.

11. Cellule électrochimique selon la revendication 10 dans laquelle la cellule électrochimique est une batterie secondaire au lithium.
